# EUROPEAN PATENT APPLICATION

(11) **EP 3 964 178 A1**
(43) Date of publication of application: **09.03.2022**
(21) Application number: 21806111.7
(22) Date of filing: 18.01.2021
(51) Int. Cl.: A61F 2/24

(54) **COMPOSITE-TYPE SKIRT FOR PROSTHETIC HEART VALVE AND PROSTHETIC HEART VALVE**

(30) Priority: 08.07.2020 CN 202010651486
(71) Applicant: Shanghai Trulive Medtech Co., Ltd, Shanghai 201206 (CN)
(72) Inventor: YANG, Wei, Shanghai 201206 (CN); ZHAO, Jing, Shanghai 201206 (CN); WEI, Lixuan, Shanghai 201206 (CN)
(74) Representative: Argyma
(86) International application number: PCT/CN2021/072480
(87) International publication number: WO 2022/007384

(57) **Abstract**

Disclosed is a composite skirt for a prosthetic heart valve. A stent includes an inflow segment, a transition segment and an outflow segment; the composite skirt includes at least two of: (1) a first skirt disposed at the inflow segment, (2) a second skirt disposed at the transition segment, and (3) a third skirt disposed at the outflow segment, wherein for the composite skirt, different skirt parameters are configured according different arrangement regions to adapt to primary tissues and reduce regurgitation. A prosthetic heart valve is further disclosed. For the composite skirt of this invention, in view of different functions in different regions of the valve, the skirt parameters of different regions are set such that the skirt is matched with a structure of the stent, thereby improving the functions of the prosthetic heart valve.

## Description

### Field of the Invention

This invention relates to the technical field of medical instruments, and more specifically, to a skirt for a prosthetic heart valve.

### Description of the Prior Art

The heart contains four heart cavities; the left atrium and left ventricle are located on the left side of the heart, and the right atrium and right ventricle are located on the right side of the heart. A ventricle inflow tract is formed between the atrium and the ventricle, the left ventricle and the aorta form the left ventricle outflow tract, and the right ventricle and the pulmonary artery form the right ventricle outflow tract. There is a valve with functions of a "one-way valve" between the ventricle inflow tract and the ventricle outflow tract to ensure the blood circulation in the heart cavity. When there is a problem with the valve, the hemodynamics of the heart change and the heart function is abnormal, resulting in a condition called a valvular heart disease.

With the development of social economy and the aging of population, the incidence of valvular heart disease has increased significantly. Studies have shown that the incidence of valvular heart disease in the population over 75 years of age is as high as 13.3%. At present, the traditional surgical treatment is still the preferred treatment for patients with severe valvular lesions. However, for patients who is older, has combined multi-organ disease, has a history of thoracotomy and has a poor heart function, the traditional surgery has disadvantages of high risk and mortality, and even no access to surgery for some patients. The transcatheter mitral valve replacement (TMVR) or the transcatheter mitral valve implantation (TMVI) has advantages of no need for thoracotomy, small trauma and quick recovery for patients, and has been widely concerned by experts and scholars.

The mitral valve is located at the left ventricle inflow tract, with a main structure of a mitral valve composite, and includes a mitral valve annulus, valve leaflets, a chordae tendineae and a papillary muscle, also including a ventricle wall in some literature. The mitral valve annulus is a dense connective tissue around a left atrioventricular orifice, wherein a front annulus consists of a part of non-coronary annuli, a part of left coronary annuli and left and right fiber triangles that are located at the aortic valve of the left ventricle outflow tract, and the rear annulus is a rear valve leaflet attachment. The front valve leaflet of the mitral valve is fiber extension of the aortic valve, which forms the left ventricle inflow tract with the rear valve leaflet and forms the left ventricle outflow tract with the heart interventricular septum. The chordae tendineae of the mitral valve is support device connecting the mitral valve leaflets with the cardiac muscle, and is distributed between the valve leaflet and the cardiac muscle, wherein subvalvular structure of the mitral valve plays an important role in maintaining the structure and functions of the left heart. A tricuspid valve, as an atrioventricular valve of the right heart, has a structure similar to that of the mitral valve, and also includes the valve leaflets, the annulus, the chordae tendineae, the papillary muscle and the cardiac muscle. Therefore, replacing primary mitral valve with heart valve prosthesis structure can also be applied to replacing a primary tricuspid valve.

In recent years, the field of mitral and tricuspid valves is developing rapidly, but there are still some difficult problems that need to be solved; for example, the overall deformation performance of the inflow region is too poor to be better adapted to the morphology of the primary tissue, the regurgitation may easily occur on the transition and outflow regions, and the thrombus is easily formed between the inter-layer of the double-layered stent, etc.

### SUMMARY OF THE INVENTION

This invention provides a composite skirt for a prosthetic heart valve, which may solve the above drawbacks in the prior art.

The technical solution of this invention is as follows:
A composite skirt for a prosthetic heart valve is provided. A stent includes an inflow segment, a transition segment and an outflow segment; the composite skirt includes at least two of: (1) a first skirt disposed at the inflow segment, (2) a second skirt disposed at the transition segment, and (3) a third skirt disposed at the outflow segment, wherein for the composite skirt, different skirt parameters are configured according to different arrangement regions to be adapted to primary tissues and to reduce the regurgitation.

The composite skirt may only include one of the first skirt, the second skirt and the third skirt. The skirt parameters should be configured according to the arrangement regions. The parameter includes elongation at break, redundancy, permeability or material of the skirt and the like to be matched with the structure of the stent, thereby improving the functions of the heart valve. Naturally, two kinds or three kinds of skirts may further be included. The skirt may be fixed to the stent by way of such as stitching, and specific arrangement regions and areas should be configured according to actual clinical needs.

The specific arrangement regions should be selected and corresponding skirt parameters are configured according to the actual clinical needs, so that each of the skirts may be matched with the function of the corresponding region while a plurality of skirts cooperating with each other, so as to achieve synergy for reducing regurgitation.

Preferably, when the composite skirt at least includes the first skirt, the elongation at break of the material of the first skirt is greater than 10%, or the redundancy greater than 10% is set when the first skirt is fixed. The first skirt has better elasticity and stretch performance, so as to be cooperated with the soft inflow segment of the stent and be better adapted to the tissues.

Preferably, when the composite skirt at least includes the second skirt, the second skirt is of a single-layered structure, and the permeability of the material of the second skirt is lower than 500 ml/cm² • min. When the skirt is of the single-layered structure, a material with lower permeability is required to reduce the regurgitation of the transition region of the valve.

Preferably, when the composite skirt at least includes the second skirt, the second skirt is of a double-layered structure, and the permeability of the material of the second skirt is smaller than 1500 ml/cm² • min. The double-layered structure can reduce the gap between the valve and the tissue, and prevent regurgitation with double layers, while the blood permeating between the double-layered structure to form a microcapsule for reducing the regurgitation. Therefore, compared with the single-layered skirt, the skirt with the double-layered structure requires a material with high permeability in case that the structures and materials of the valve are the same.

Preferably, this invention further includes the third skirt, wherein the permeability of the material of the second skirt is greater than a permeability of a material of the third skirt, and the permeability of the material of the third skirt is lower than 500 ml/cm² • min, which may effectively reduce the regurgitation.

Preferably, when the composite skirt at least includes the third skirt, and the permeability of the material of the third skirt is lower than 500 ml/cm² • min. Thus, with the function of reducing blood permeation, the regurgitation is reduced.

When the composite skirt at least includes the first skirt, the first skirt covers an atrium-end edge of the inflow segment of the stent in order to prevent the human tissues from being damaged by sharp stent rods during the implantation process. Or, when the composite skirt at least includes the third skirt, the third skirt covers a ventricle-end edge of the outflow segment of the stent. Both the atrium-end edge and the ventricle-end edge of the stent are covered by the skirt to avoid the tissues from being damaged by the bare stent.

Preferably, when the stent is of an internal-external double-layered structure, the stent forms a ring-shaped region; and the composite skirt further comprises a fourth skirt disposed in the ring-shaped region, and the fourth skirt is made from an antithrombotic material, allowing blood to flow into and out of the ring-shaped region to prevent the formation of thrombus in the ring-shaped region. Specifically, the material of the fourth skirt may be a polyurethane-heparin graft copolymer, a polyion complex or a combination thereof.

Preferably, when the stent is of the internal-external double-layered structure, the stent forms a ring-shaped region, the ring-shaped region has an enclosed end axially along the heart valve, and the composite skirt further includes the fourth skirt; the fourth skirt is disposed on an outer surface of an inner stent, on an inner surface of an outer stent and above the enclosed end, to form an enclosed skirt region, and the material of the fourth skirt allows the blood to enter the enclosed skirt region but prevents the formed thrombus from leaving. The fourth skirt may block and/or decelerate the blood flow in the enclosed skirt region, reduce the power flushing of the blood, and increase the thrombus formed in the enclosed skirt region, as well as blocking the outflow of the thrombus to form a pot enclosure, thereby further stabilizing the valve prosthesis.

Specifically, the material of the fourth skirt may be selected from a knitted, tatted and woven polyester fabric, PTFE, ePTFE, a bio-tissue material or a combination thereof.

This invention further provides a prosthetic heart valve including the composite skirt according to any one of the above descriptions.

Compared with the conventional art, this invention has the following beneficial effects:
For the composite skirt of this invention, different parameters of the first skirt, the second skirt and the third skirt are configured according to the arrangement regions to be matched with the structure of the stent for improving the function of the heart valve, wherein the first skirt disposed in the inflow region has better elasticity and stretch performance so as to be cooperated with the soft inflow segment of the stent and be better adapted to the tissues; the corresponding permeability is selected for the material of the second skirt and the material of the third skirt, which may reduce the regurgitation; when the permeability of the second skirt is greater than the permeability of the third skirt, the regurgitation is further reduced; when the stent is of the internal-external double-layered structure, by disposing the fourth skirt and configuring the same with the antithrombotic material, the thrombus may be effectively prevented from being formed or the blood is allowed to flow in while blocking the thrombus from flowing out for forming the pot enclosure.

Certainly, any one product for implementing this invention is unnecessary to achieve all the above advantages at the same time.

### BRIEF DESCRIPTION OF THE DRAWINGS

Fig. 1 is a view of a prosthetic heart valve in an embodiment of this invention;
Fig. 2 is a view of a heart valve with a double-layered stent in an embodiment of this invention;
Fig. 3 is another view of a heart valve with a double-layered stent in an embodiment of this invention.

Reference for numerals: valve prosthesis-100; stent-110; skirt-120; valve prosthetic leaflet-130; inflow region-101; transition region-102; outflow region-103; inflow segment-Ill; transition segment-112; outflow segment-113; first skirt-A; second skirt-B; third skirt-C; fourth skirt-D; valve prosthesis-200; stent-210; valve skirt-220; valve inflow region-201; valve transition region-202; valve outflow region-203; valve inflow segment-211; valve transition segment-212; valve outflow segment-213; inner stent-2101; outer stent-2102.

### DESCRIPTION OF THE PREFERRED EMBODIMENTS

This invention provides a composite skirt for a prosthetic heart valve. Through the design of the material of the skirt in different regions of the valve prosthesis, problems of poorer overall deformation performance of the inflow region and of being prone to occur the regurgitation on the transition region and the outflow region are solved, while solving the problems such as thrombosis easily occurs between the inter-layer of the double-layered stent, thereby improving the functions of the heart valve.

Taking a mitral valve prosthesis as an example, the following content will describe the composite skirt of this invention.

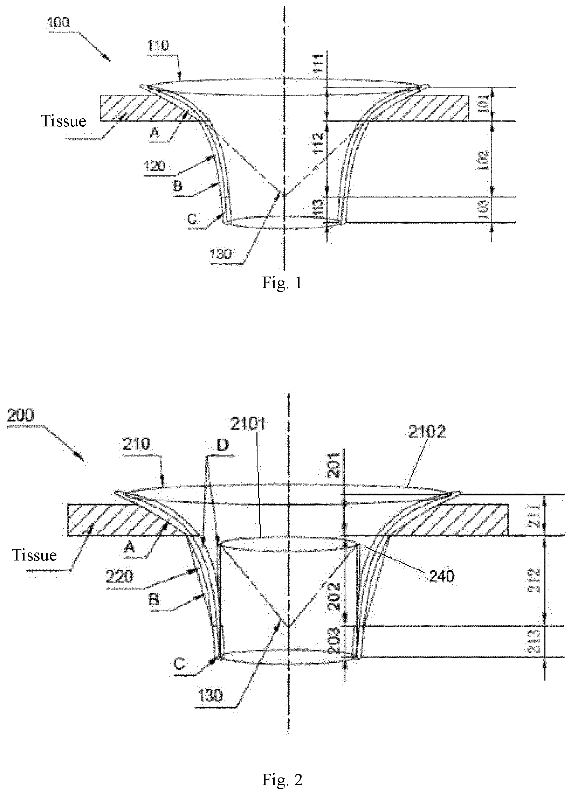

As shown in Fig. 1, the mitral valve prosthesis 100 consists of a stent 110, a skirt 120 and a valve prosthetic leaflet 130, and may be divided into an inflow region 101, a transition region 102 and an outflow region 103 in a longitudinal direction. After the mitral valve prosthesis 100 is implanted into a human body, the inflow region 101 is attached to a primary mitral valve annulus of the heart to prevent the valve prosthesis from falling from the left atrium into the left ventricle, and the transition region 102 is used to carry the valve prosthetic leaflet 130 while being supported on the tissue by the anchoring force of the stent for functioning as an anchor and a seal.

Corresponding to the valve prosthesis 100, the stent 110 is divided into an inflow segment 111, a transition segment 112 and an outflow segment 113. The stent may provide the valve prosthesis 100 with several functions, which include functioning as a main body structure and an anchoring structure (including an anchoring claw structure grabbing the valve leaflets or puncturing into the valve leaflets, and so on), carrying a support member of the internal valve prosthetic leaflet 130, functioning as a sealing member that inhibits the regurgitation of the mitral valve prosthesis 100 and a structure (a hanging tab or a fixing tab) connected to a transporting system, and so on.

The stent 110 may adopt such as nitinol, titanium alloy, cobalt chromium alloy, MP35n, 316 stainless steel, L605, Phynox/Elgiloy and platinum chromium, or bio-compatible metal frame or laser-cut solid metal tube made from other bio-compatible metals known by those skilled in the art. Preferably, the stent may be prepared by a shape memory alloy, and optionally may also include an elastic or malleable deformable material such as a balloon-expandable material, or may be a shape memory alloy that may respond to temperature changes to convert between a collapsed delivery state and an expanded expansion state. Optionally, the stent 110 may also be constructed by weaving threads or other suitable materials.

The valve prosthetic leaflet 130 dynamically switches between two states of an opening state and a closing state. When the valve prosthetic leaflet 130 is in the closing state, the valve prosthetic leaflet 130 closes by way of sealing and abutting. The valve prosthetic leaflet 130 may be formed by any suitable materials or a combination of materials, and may, in some embodiments, be prepared by biological tissues such as chemically stable tissues from the heart valves of animals such as pigs or pericardial tissues such as cattle (bovine pericardial tissues) or sheep (ovine pericardial tissues) or pigs (porcine pericardial tissues) or horses (equine pericardial tissues), preferably the bovine pericardial tissues, or may be also prepared by small intestinal submucosa tissues.

In some embodiments, the valve prosthetic leaflet 130 may be prepared by synthetic materials, such as an expanded PTFE or a polyester. Optionally, the material further includes thermoplastic polyurethane polyester, polyether urethane, segmented polyether urethane, silicone polyether urethane, silicone-polyurethane polyester, and ultra-high molecular weight polyethylene.

In some embodiments, the valve prosthetic leaflet 130 may further be prepared by bio-compatible polymers. The bio-compatible polymer optionally may include polyolefin, elastomer, polyethylene glycol, polyethersulfone, polysulfone, polyvinylpyrrolidone, polyvinyl chloride, other fluorine-containing polymers, silicone polyester, siloxane polymers and/or olimers, and/or polycaprolactone, and block copolymers thereof.

In some embodiments, the valve leaflet 130 further has a surface treated (or reacted with) by anticoagulants, and the anticoagulant includes but is not limited to heparinized polymers.

The stent 110 is disposed with the composite skirt. The composite skirt may be of a single-layered structure disposed on an inner surface of the stent 110 or disposed on an outer surface of the stent 110; the skirt may also be of a double-layered structure disposed on the inner surface and the outer surface of the stent 110 simultaneously, or the skirt with the double-layered structure is directly disposed on the outer surface of the stent 110. Since different parts of the valve have different structural features with corresponding different functions, different designs for the skirt may be adopted to ensure the sealing of each of the skirts.

Specifically, the first skirt requires a certain degree of ductility and malleability, which may be achieved by the selection of materials or through the configuration of structure, e.g., by selecting a material that has a certain degree of elasticity or configuring a certain amount of redundancy (e.g. folds). This design has the following purposes: 1. Since the tissue around the annulus of the patient does not have a regular surface and the heart valve is also not of a regular shape, the first skirt is required to have a certain degree of ductility and malleability so that the inflow region of the heart valve fits as closely as possible the tissue around the annulus through the first skirt for improving the sealing performance; 2. The heart valve is anchored at the annulus, and the heart valve may move slightly as the heart moves (shrinks or dilates); since the tissue around the annulus does not necessarily have the regular (e.g. uneven) surface, the first skirt is required to have a certain degree of ductility and malleability to fit closely the tissue, so as to improve the sealing performance; 3. The heart valve will be mass produced to be used as a standardized part, and with the consideration of the individual difference, the first skirt is required to have a certain degree of ductility and malleability in order to be applied to different individuals; 4. During the heart activity (shrinkage or dilation), the stent of the heart valve may be deformed slightly; therefore, the first skirt is required to have a certain degree of ductility and malleability in order to prevent possible folds from the stent's deformation, thereby improving the sealing performance.

Through the selection of the permeability for the second skirt and the third skirt, the regurgitation is caused to be smaller than 25%; when the permeability of the second skirt is greater than the permeability of the third skirt, the regurgitation is further reduced.

The inflow region 101 of the mitral valve prosthesis 100 is required to be well adapted to the morphology of the primary tissue. Therefore, the inflow region 101 is disposed with a first skirt A, and the first skirt A is required to have better elasticity. The first skirt A is located at the stent inflow segment 111, and covers an outer layer of the stent inflow segment 111; preferably, the first skirt A folds from the outer layer of the stent inflow segment 111 to an inner layer of the stent inflow segment 111. The skirt material of the first skirt A is a first skirt material, the first skirt material is required to have better stretch performance, and the first skirt A is matched with the soft stent inflow segment 111 to be better adapted to the tissues. In an embodiment, the elongation at break of the material of the first skirt is greater than 10%, preferably between 10% and 400%; when the elongation at break is too small, the stent inflow segment 111 is difficult to be better adapted to the primary tissue; when the elongation at break is too large, the material is easily deformed to cause accumulation of the material.

In some embodiments, the first skirt A may be designed to have a redundancy of greater than 10% for realizing elasticity and being better adapted to the tissue, e.g., by adopting multi-layer folding stitching, and then there is no requirement for the elongation at break of the first skirt material itself. The first skirt material may be selected from a knitted PET.

The regurgitation may easily occur in the transition region 102 of the mitral valve prosthesis 100. The transition region is disposed with a second skirt B, the second skirt B covers the stent transition segment 112, and the second skirt B may effectively reduce the regurgitation. A skirt material of the second skirt is a second skirt material, and the material of the second skirt may be selected from materials such as a knitted, tatted and woven polyester fabric, PTFE, or ePTFE.

When the second skirt B is of the single-layered structure, an inner surface or an outer surface of the stent transition segment 112 may be covered by the second skirt B, and the permeability of the second skirt material is lower than 500 ml/cm²-min ( permeability under a pressure of 100~140mmHg). This is because the second skirt B being the single-layered structure requires a material with lower permeability to reduce the regurgitation.

When the second skirt B is of the double-layered structure, the inner surface and the outer surface of the stent transition segment 112 may be covered respectively by the second skirt B or the skirt of the double-layered structure covers the outer surface of the stent transition segment 112, and then the permeability of the second skirt material is required to be lower than 1500 ml/cm² · min (permeability under a pressure of 100~140mmHg). This is because the second skirt B is of an internal-external double-layered structure, which may reduce the gap between the valve and the tissue, and prevent the regurgitation with double layers while the blood permeating between the double-layered structure to form a microcapsule for reducing the regurgitation. Therefore, compared with the single-layered skirt, the skirt with the double-layered structure requires the material with a greater permeability in case that other structures and materials of the valve are the same.

The regurgitation may easily occur in the outflow region 103 of the mitral valve prosthesis 100, and the outflow region 103 is disposed with a third skirt C to effectively prevent the regurgitation. The third skirt C covers an outer layer of the stent outflow segment 113, and preferably, the third skirt C further folds from the outer layer of the stent outflow segment 113 to the inner layer of the stent outflow segment 113 to form the double-layered structure. During the process of implanting the stent, the folding structure may prevent the human tissues from being damaged by sharp stent rods. Naturally, in other embodiments, the third skirt C may also fold from the inner layer of the stent outflow segment 113 to the outer layer thereof.

Specifically, a skirt material of the third skirt C is a third skirt material, and the permeability of the third skirt material is required to be lower than 500 ml/cm² · min (permeability under a pressure of 100~140mmHg), preferably lower than 300 ml/cm² ( permeability under a pressure of 100~140mmHg); the third skirt material may be a knitted, tatted and woven polyester fabric, PTFE, ePTFE, a bio-tissue material and the like with functions of reducing the permeation of the blood, thereby reducing the regurgitation.

Since the size of the annulus of the mitral valve is anatomically larger and a part of the support body of the valve prosthesis implanted with the mitral valve used to carry the prosthetic valve requires a larger size in either a circumferential diameter or an axial height, the size of the subvalvular prosthesis structure is larger after the valve prosthesis is implanted into the mitral valve, which causes a greater risk in damage on the subvalvular structure of the primary valve composition. At the same time, the subvalvular prosthesis structure is too large, which may affects the ejection function of the aorta and cause blockage of the left ventricle outflow tract. For a part of patients with mitral regurgitation, there is no calcification part on the valve, and the existing working principle of using the existing radial support force generated between the valve prosthesis and the primary valve to prevent the valve prosthesis from shifting may not be applied, so the valve prosthesis with the double-layered stent has a better treating effect. The double-layered mitral valve prosthesis may assign the functions of carrying the valve prosthetic leaflet, anchoring and sealing to different single-layered valve components, so as to achieve the purpose of not affecting the normal operation of other structures of the heart and better playing the function of implantation treatment.

With reference to Fig. 2, another mitral valve prosthesis is shown. The mitral valve prosthesis 200 consists of a stent 210, a valve skirt 220 and an valve prosthetic leaflet 130, wherein the stent 210 is of the internal-external double-layered structure, including an inner stent 2101 and an outer stent 2102. The stent may be divided into a valve inflow region 201, a valve transition region 202 and a valve outflow region 203 in the longitudinal direction. A subvalvular height of the valve prosthesis in the axial direction is smaller as compared to the traditional mitral valve prosthesis, which may not cause adverse effects on the subvalvular tissues.

Corresponding to the valve prosthesis 200, the stent 210 is divided into a valve inflow segment 211, a valve transition segment 212 and a valve outflow segment 213. The internal-external double-layered structure of the stent 210 may provide the valve prosthesis 200 with different functions. The inner stent 2101 is a support member for carrying the inner valve prosthetic leaflet 130, and the outer stent 2102 may serve as the anchoring structure (including an anchoring claw structure grabbing the valve leaflets or puncturing into the valve leaflet, and so on), a sealing member for inhibiting the regurgitation of the mitral valve prosthesis 200 and a structure (a hanging tab or a fixing tab) connected to a delivery system, and so on. A ring-shaped region 240 is formed between the inner stent 2101 and the outer stent 2102. When the valve prosthesis 200 is placed into the annulus of the human heart valve, the blood from the atrium may flow into and flow out from the ring-shaped region 240 between the inner stent 2101 and the outer stent 2102. The blood can be clotted to cause the thrombosis, which can be delivered through the blood flow during the heart's circulatory pumping, causing blockages in blood vessels, which in severe cases can cause cerebral thrombosis and even life-threatening.

Therefore, in an embodiment, the outer surface of the outer stent 2102 is disposed with the first skirt A, the second skirt B and the third skirt C respectively from the inflow region to the outflow region, and the composite skirt further includes a fourth skirt D disposed in the ring-shaped region 240, the fourth skirt D being made from the antithrombotic skirt material. The fourth skirt D is attached to the outer layer of the inner stent 2101 and the inner layer of the outer stent 2102; the skirt material of the fourth skirt D has the antithrombotic function, and optionally is a polyurethane-heparin graft copolymer, a polyion complex and the like; preferably, the skirt material of the fourth skirt D is the polyurethane-heparin graft copolymer.

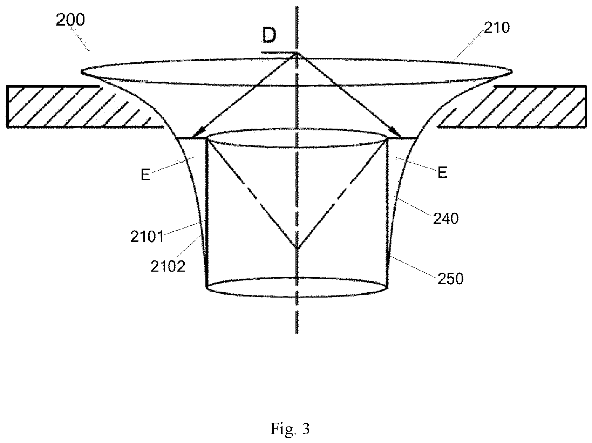

For the double-layered valve of different types, the ring-shaped region 240 of the inner and outer layer stents adopts different skirt materials. Studies have shown that the ring-shaped region 240 filled with a large number of thrombosis may be used as a pot seal for inner layer structure of the valve prosthesis (including the inner stent, and the skirt and the valve prosthetic leaflet thereof), so as to further stabilize the valve prosthesis. As shown in Fig. 3, if the ring-shaped region 240 is of an enclosed structure, this type of valve controls the risk by blocking the outflow of the thrombosis with the skirt, which has a mechanism that is different from the above-mentioned antithrombotic mechanism.

As shown in Fig. 3, the atrium end of the stent 210 is an enclosed end 250, and the ring-shaped region 240 is disposed with the fourth skirt D; the fourth skirt D is attached to the outer layer of the inner stent 2101, the inner layer of the outer stent 2102 and the enclosed end 250 to enclose the ring-shaped region 240 entirely for forming an enclosed skirt region E. The fourth skirt D adopts a skirt material of D2 type; the skirt material of D2 type should block and/or decelerate the blood flow in the skirt region E, reduce the power flushing of the blood, and increase the thrombosis formed in the enclosed skirt region. Specifically, the skirt material of D2 type allows the blood (in particular, including erythrocytes) to enter the enclosed skirt region E but prevents large thrombosis from leaving the region. The D2 material is the knitted, tatted and woven polyester fabric, PTFE, ePTFE, and so on.

As mentioned here, the skirt materials of the first skirt A, the second skirt B, the third skirt C and the fourth skirt D may be the same raw material, which may be prepared by different process means, and also may be prepared by different raw materials.

As mentioned here, the above composite skirt similarly is suitable for the tricuspid valve, the pulmonary valve and the aortic valve, and adaptive adjustments may be made to the skirt material according to the specific structure and the implantation location.

In the description of this invention, a range represented by "one value to another value" is a way to avoid listing in the specification a summary representation of all values in that range. Therefore, a record of a particular range of values covers any number within that range and a small range of values defined by any number within that range, which is the same as the arbitrary value and the smaller range of values written clearly in the specification.

In the description of this invention, it should be noted that "valve," "valve prosthesis," "valve prosthesis," and "prosthetic heart valve" have the same meaning.

In the description of this invention, it should be noted that orientations or position relationships indicated by terms "center", "upper", "lower", "left", "right", "vertical", "horizontal", "inside", "outside" and the like are orientations or position relationships shown in the drawings, and these terms are merely for facilitating description of this invention and simplifying the description, but not for indicating or implying that the mentioned device or elements must have a specific orientation and must be established and operated in a specific orientation, and thus, these terms cannot be understood as a limitation to this invention. Moreover, terms like "first", "second", "third" etc. are only used for description, not be considered as a designation or designation of relative importance.

In the description of this invention, singular forms "a/an," "one," and "the/that" include plural objects, unless otherwise explicitly stated. As used in the specification, the term "or" is usually used to include the meaning of "and/or", unless otherwise expressly stated.

The following further describes this invention in combination with specific embodiments.

It should be noted that in the following embodiments, the stents used are all single-layered stents, the second skirt is disposed at the transition segment and the third skirt is disposed at the outflow segment. In the following embodiments, the second skirt and the third skirt are made from the PET as the raw material, and different permeabilities are generated from different process means.

### Embodiment 1

The present embodiment provides a composite skirt, which includes the second skirt and the third skirt, the permeability of the second skirt material is indicated by B', and the permeability of the third skirt material is indicated by C'.

The skirt is connected to the stent for test, and both the second skirt and the third skirt are of single-layered structures and are fixed at the outer surface of the stent respectively; an MPD-1000 in vitro test instrument (Suzhou Heartpartner Testing Equipment Co., Ltd) is used, according to a YY1449.3-2016 method, for a regurgitation test under conditions that the heart rate is 70 bpm, the simulated cardiac output is 5.0L/min, the average aortic pressure is 100mmHg and the cardiac shrinking percentage is 35%, and the test results are accepted according to the YY/T 1449.3-2016 standard (regurgitation smaller than 25%); the test results are shown in Table 1.

**Table 1 Effects on valve regurgitation from skirt material of second skirt and third skirt**

| No. | B'/(ml/cm²• min) | C' /(ml/cm² • min) | regurgitation | if standard met or not? |
|---|---|---|---|---|
| 1 | 420 | 410 | 17% | yes |
| 2 | 460 | 410 | 21% | yes |
| 3 | 480 | 410 | 23% | yes |
| 4 | 520 | 410 | 27% | no |
| 5 | 600 | 410 | 32% | no |

From Table 1, it can be known that when the permeability C' of the third skirt material remains unchanged and the permeability B' of the second skirt material increases to 520 ml/cm²·min, the valve regurgitation exceeds 25%, which does not meet the standard. Therefore, when the second skirt is of the single-layered structure, the permeability B' of the material of the second skirt is preferably lower than 500 ml/cm²·min.

### Embodiment 2

The present embodiment provides a composite skirt, which includes the second skirt and the third skirt, the permeability of the second skirt material is indicated by B', and the permeability of the third skirt material is indicated by C'.

The skirt is connected on the stent for the test, wherein the second skirt is of the double-layered structure and fixed on the inner surface and the outer surface of the stent transition segment, and the third skirt is of the single-layered structure fixed on the outer surface of the stent. The method of Embodiment 1 is used for the regurgitation test, and the results are shown in Table 2.

**Table 2 Effects on valve regurgitation from skirt material of second skirt and third skirt**

| No. | B'/(ml/cm²• min) | C' /(ml/cm² • min) | regurgitation | if standard met or not? |
|---|---|---|---|---|
| 1 | 850 | 410 | 16% | yes |
| 2 | 1100 | 410 | 19% | yes |
| 3 | 1450 | 410 | 24% | yes |
| 4 | 1550 | 410 | 26% | no |
| 5 | 1680 | 410 | 30% | no |

From Table 2, it can be known that when C' remains unchanged and B' increases to 1550 ml/cm² · min gradually, the valve regurgitation exceeds 25%, which does not meet the standard. Therefore, when the second skirt is of the double-layered structure, the permeability B' of the material of the second skirt is preferably lower than 1500 ml/cm²·min.

### Embodiment 3

The present embodiment provides a composite skirt, which includes the second skirt and the third skirt, the permeability of the second skirt material is indicated by B', and the permeability of the third skirt material is indicated by C'.

The skirt is connected on the stent for the test, wherein both the second skirt and the third skirt are of the single-layered structures, which are fixed on the outer surface of the stent respectively. The method of Embodiment 1 is used for the regurgitation test, and the results are shown in Table 3.

**Table 3 Effects on valve regurgitation from third skirt when skirt material of second skirt remains unchanged**

| No. | B'/(ml/cm²• min) | C' /(ml/cm² • min) | regurgitation | if standard met or not? |
|---|---|---|---|---|
| 1 | 420 | 300 | 7% | yes |
| 2 | 420 | 400 | 13% | yes |
| 3 | 420 | 470 | 17% | yes |
| 4 | 420 | 490 | 21% | yes |
| 5 | 420 | 520 | 26% | no |
| 6 | 420 | 590 | 29% | no |

From Table 3, it can be known that when the permeability B' of the second skirt material remains unchanged and C' increases to 520 ml/cm²· min gradually, the valve regurgitation exceeds 25%, which does not meet the standard. Therefore, the permeability C' of the third skirt material is preferably lower than 500 ml/cm²·min, and further preferably, C' is lower than 300 ml/cm²·min.

### Embodiment 4

The present embodiment provides a composite skirt, which includes the second skirt and the third skirt, the permeability of the second skirt material is indicated by B', and the permeability of the third skirt material is indicated by C'.

The skirt is connected on the stent for the test, wherein the second skirt is of the double-layered structure and fixed on the inner surface and the outer surface of the stent, and the third skirt is of the single-layered structure fixed on the outer surface of the stent. The method of Embodiment 1 is used for the regurgitation test, and the results are shown in Table 4.

**Table 4 Effects on valve regurgitation from third skirt when skirt material of second skirt remains unchanged**

| No. | B'/(ml/cm²• min) | C' /(ml/cm² • min) | regurgitation | if standard met or not? |
|---|---|---|---|---|
| 1 | 820 | 300 | 7% | yes |
| 2 | 820 | 400 | 14% | yes |
| 3 | 820 | 470 | 17% | yes |
| 4 | 820 | 490 | 21% | yes |
| 5 | 820 | 520 | 26% | no |
| 6 | 820 | 590 | 29% | no |

From Table 4, it can be known that when the permeability B' of the second skirt material remains unchanged and C' is increased to 520 ml/cm²·min gradually, the valve regurgitation exceeds 25%, which does not meet the standard. Therefore, the permeability C' of the third skirt material is preferably lower than 500 ml/cm²·min, and further preferably, C' is lower r than 300 ml/cm²·min.

### Embodiment 5

The present embodiment provides a composite skirt, which includes the second skirt and the third skirt, the permeability of the second skirt material is indicated by B', and the permeability of the third skirt material is indicated by C'.

The skirt is connected on the stent for the test, wherein both the second skirt and the third skirt are of the single-layered structures, which are fixed on the outer surface of the stent respectively. The method of Embodiment 1 is used for the regurgitation test, and the results are shown in Table 5.

**Table 5 Effects on valve regurgitation from skirt material of second skirt and third skirt**

| No. | B' /(ml/cm² • min) | C' /(ml/cm² • min) | regurgitation |
|---|---|---|---|
| 1 | 420 | 420 | 15% |
| 2 | 420 | 470 | 17% |
| 3 | 420 | 300 | 7% |

From Table 5, it can be known that when the permeability B' of the second skirt material is higher than the permeability C' of the third skirt material, the regurgitation may be effectively reduced.

### Embodiment 6

The present embodiment provides a composite skirt, which includes the second skirt and the third skirt, the permeability of the second skirt material is indicated by B', and the permeability of the third skirt material is indicated by C'.

The skirt is connected on the stent for the test, wherein the second skirt is of the double-layered structure and fixed on the inner surface and the outer surface of the stent, and the third skirt is of the single-layered structure fixed on the outer surface of the stent. The method of Embodiment 1 is used for the regurgitation test, and the results are shown in Table 6.

**Table 6 Effects on valve regurgitation from skirt material of second skirt and third skirt**

| No. | B' /(ml/cm² • min) | C' /(ml/cm² • min) | regurgitation |
|---|---|---|---|
| 1 | 850 | 850 | 35% |
| 2 | 850 | 1000 | 41% |
| 3 | 850 | 720 | 32% |

From Table 6, it can be known that when the permeability B' of the second skirt material is higher than the permeability C' of the third skirt material, the regurgitation may be effectively reduced.

Test method for permeability:
Step 1: the test material is fixed between an upper hollow cylinders and a lower hollow cylinders;
Step 2: a certain degree of fluid pressure is exerted on one ends of the hollow cylinders, and the permeability at the other ends over a period of time is recorded for the test material.

The above disclosure is only the preferred embodiment of this invention. The preferred embodiments do not describe all the details, and are not intended to limit the invention only to be the specific embodiments. It is obvious that various modifications and changes can be made to the content of the specification. This invention selects and specifically describe the embodiments with the purpose of better explain the principle and practical use of this invention, such that a person skilled in the art can well utilize this invention. This invention is merely limited by the appended claims and the scope and equivalents thereof.

## Claims

1. A composite skirt for an prosthetic heart valve, wherein a stent comprises an inflow segment, a transition segment and an outflow segment, the composite skirt comprising at least two of (1) a first skirt disposed at the inflow segment, (2) a second skirt disposed at the transition segment, and (3) a third skirt disposed at the outflow segment, wherein for the composite skirt, different skirt parameters are configured according different arrangement regions to adapt to primary tissues and reduce regurgitation.

2. The composite skirt for a prosthetic heart valve according to claim 1, wherein when the composite skirt at least comprises the first skirt, an elongation at break of a material of the first skirt is greater than 10%, or a redundancy greater than 10% is set when the first skirt is fixed.

3. The composite skirt for an prosthetic heart valve according to claim 1, wherein when the composite skirt at least comprises the second skirt, the second skirt is of a single-layered structure, and the permeability of a material of the second skirt is lower than 500 ml/cm²·min.

4. The composite skirt for an prosthetic heart valve according to claim 1, wherein when the composite skirt at least comprises the second skirt, the second skirt is of a double-layered structure, and the permeability of the material of the second skirt is lower than 1500 ml/cm²·min.

5. The composite skirt for an prosthetic heart valve according to claim 3 or 4, further comprising the third skirt, wherein the permeability of the material of the second skirt is greater than the permeability of the material of the third skirt, and the permeability of the material of the third skirt is lower than 500 ml/cm²·min.

6. The composite skirt for an prosthetic heart valve according to claim 1, wherein when the composite skirt at least comprises the third skirt, the permeability of the material of the third skirt is lower than 500 ml/cm²· min.

7. The composite skirt for an prosthetic heart valve according to claim 1, wherein when the composite skirt at least comprises the first skirt, the first skirt covers an atrium-end edge of the inflow segment of the stent; or when the composite skirt at least comprises the third skirt, the third skirt covers a ventricle-end edge of the outflow segment of the stent.

8. The composite skirt for an prosthetic heart valve according to claim 1, wherein when the stent is of an internal-external double-layered structure, the stent forms a ring-shaped region; and the composite skirt further comprises a fourth skirt disposed in the ring-shaped region, and the fourth skirt is made from an antithrombotic material to prevent thrombosis in the ring-shaped region.

9. The composite skirt for a prosthetic heart valve according to claim 8, wherein a material of the fourth skirt is a polyurethane-heparin graft copolymer, a polyion complex or a combination thereof.

10. The composite skirt for a prosthetic heart valve according to claim 1, wherein when the stent is of the internal-external double-layered structure, the stent forms a ring-shaped region, the ring-shaped region has an enclosed end axially along the heart valve, and the composite skirt further comprises the fourth skirt; the fourth skirt is disposed on an outer surface of an inner stent, an inner surface of an outer stent and above the enclosed end to form an enclosed skirt region, and the material of the fourth skirt allows blood to enter the enclosed skirt region but prevents formed thrombus from leaving.

11. The composite skirt for a prosthetic heart valve according to claim 10, wherein the material of the fourth skirt is a knitted, tatted or woven polyester fabric, PTFE, ePTFE, a bio-tissue material or a combination thereof.

12. A prosthetic heart valve comprising the composite skirt according to any one of claims 1 to 11.
